(19) Europäisches Patentamt
European Patent Office
Office européen des brevets

(11) Veröffentlichungsnummer: **0 120 448**
**A1**

(12) # EUROPÄISCHE PATENTANMELDUNG

(21) Anmeldenummer: 84103083.6

(22) Anmeldetag: 21.03.84

(51) Int. Cl.³: **C 07 D 213/70**
A 01 N 43/34, A 01 N 43/38
A 01 N 43/72, C 07 D 215/22
C 07 D 215/36, C 07 D 213/64
C 07 D 213/69, C 07 D 211/74
C 07 D 231/26, C 07 D 231/20

(30) Priorität: 23.03.83 DE 3310418

(43) Veröffentlichungstag der Anmeldung:
03.10.84 Patentblatt 84/40

(84) Benannte Vertragsstaaten:
CH DE FR GB IT LI

(71) Anmelder: BASF Aktiengesellschaft
Carl-Bosch-Strasse 38
D-6700 Ludwigshafen(DE)

(72) Erfinder: Jahn, Dieter, Dr.
Burgunder Weg 8
D-6803 Edingen-Neckarhausen(DE)

(72) Erfinder: Becker, Rainer, Dr.
Im Haseneck 22
D-6702 Bad Duerkheim(DE)

(72) Erfinder: Keil, Michael, Dr.
Fontanestrasse 4
D-6713 Freinsheim(DE)

(72) Erfinder: Spiegler, Wolfgang, Dr.
Westpreussenstrasse 5
D-6520 Worms 27(DE)

(72) Erfinder: Wuerzer, Bruno, Dr. Dipl.-Landwirt
Ruedigerstrasse 13
D-6701 Otterstadt(DE)

(54) **Cyclohexan-1,3-dion-derivate, Verfahren zu ihrer Herstellung und ihre Verwendung zur Bekämpfung unerwünschten Pflanzenwuchses.**

(57) Die Erfindung betrifft Cyclohexan-1, 3-dionderivate der Formel

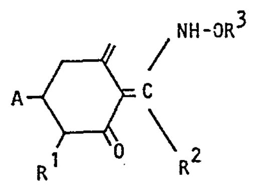

in der
A einen gegebenenfalls ungesättigten, gegebenenfalls substituierten 4-bis 7-gliedrigen Heterocyclus mit 1 bis 3 Stickstoffatomen, der mit einem Aromaten kondensiert sein kann,
$R^1$ Wasserstoff, Methoxycarbonyl, Ethoxycarbonyl, Methyl oder Cyano,
$R^2$ Alkyl und
$R^3$ Alkyl, Halogenalkenyl oder Propargyl bedeuten,
und Salze dieser Verbindungen, Verfahren zu ihrer Herstellung und ihre Verwendung zur Bekämpfung unerwünschten Pflanzenwuchses.

EP 0 120 448 A1

Cyclohexan-1,3-dion-derivate, Verfahren zu ihrer Herstellung und ihre Verwendung zur Bekämpfung unerwünschten Pflanzenwuchses

Die Erfindung betrifft Cyclohexan-1,3-dion-derivate, Verfahren zu ihrer Herstellung, sowie Herbizide, die diese Verbindungen als Wirkstoff enthalten.

Es ist bekannt, Cyclohexan-1,3-dion-derivate zur Bekämpfung von unerwünschten Gräsern in breitblättrigen Kulturen zu verwenden (DE-OS 24 39 104).

Es wurde gefunden, daß Cyclohexan-1,3-dion-derivate der Formel

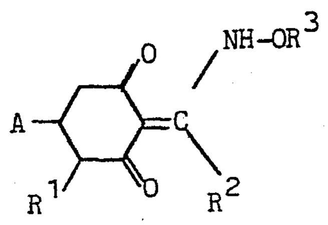

(I),

in der

A einen 4- bis 7-gliedrigen Heterocyclus mit 1 bis 3 Stickstoffatomen, der eine oder zwei Oxo- oder Thioxogruppen enthält und der gegebenenfalls ungesättigt, mit einem Aromaten kondensiert, mit maximal 3 Alkylresten mit bis zu 4 C-Atomen, dem Hydroxyrest, einem Alkoxyrest mit bis zu 4 C-Atomen oder einem gegebenenfalls substituierten Phenylrest substituiert sein kann,

$R^1$ Wasserstoff, Methoxycarbonyl, Ethoxycarbonyl, Methyl oder Cyano,

$R^2$ Alkyl mit 1 bis 4 C-Atomen und

$R^3$ Alkyl mit 1 bis 3 C-Atomen, Alkenyl mit 3 oder 4 C-Atomen, Halogenalkenyl mit 3 oder 4 C-Atomen und 1 bis 3 Halogensubstituenten oder Propargyl bedeuten,

und Salze dieser Verbindungen

gegen Gräser herbizid wirkam sind und sowohl breitblättrige Kulturpflanzen und monokotyle Kulturen, welche nicht zur Familie der Gräser (Gramineen) zählen, sowie Weizen nicht oder nur wenig schädigen.

H/HB

Die Verbindungen der Formel I können in mehreren tautomeren Formen auftreten, die alle vom Patentanspruch umfaßt werden:

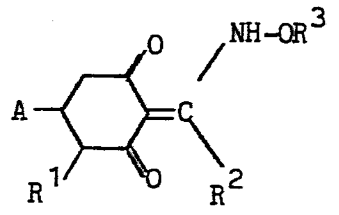

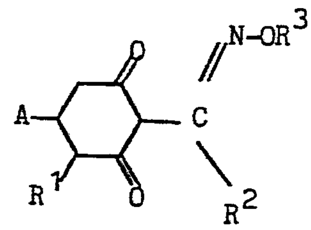

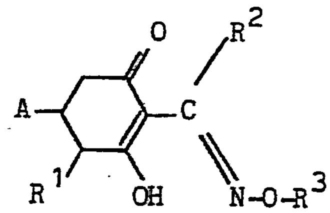

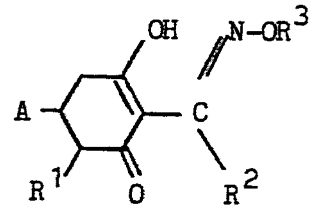

A in Formel I bedeutet einen 4- bis 7-gliedrigen Heterocyclus mit 1 bis 3 Stickstoffatomen, der eine oder zwei Oxo- oder Thioxogruppen enthält und der gegebenenfalls ungesättigt, mit einem Aromaten kondensiert, mit maximal 3 Alkylresten mit bis zu 4 C-Atomen, einem Hydroxyrest, einem Alkoxyrest mit bis zu 4 C-Atomen oder einem gegebenenfalls substituierten Phenylrest substituiert sein kann. Beispiele für A sind:

gegebenenfalls substituierte 2(1H)-Pyridonylreste, 2(1H)-Pyridinthionylreste, Piperidonylreste, Oxochinolinreste, Thioxochinolinreste, Pyrazolinonylreste, Oxobenzoxazinylreste, Oxobenzthiazinylreste, Oxoindolinylreste sowie Pyridinylreste, insbesondere 2(1H)-Pyridon-2-yl, 1-Methyl--2(1H)-pyridon-3-yl, 1-Phenyl-2(1H)-pyridon-3-yl, 1-tert.-Butyl-2(1H)--pyridon-3-yl, 2-(1H)-Pyridinthion-3-yl, 1-Methyl-2(1H)-pyridinthion-3--yl, 1-Phenyl-2(1H)-pyridinthion-3-yl, 1-tert.-Butyl-2-(1H)-pyridinthion--3-yl, 6-Methyl-2(1H)-pyridon-3-yl, 4,6-Dimethyl-2(1H)-pyridon-3-yl, 4-Hydroxy-6-methyl-2(1H)-pyridon-3-yl, 1-Methyl-4-methoxy-2(1H)-pyridon--3-yl, 1-Ethyl-4-ethoxy-2(1H)-pyridon-3-yl, 2-Piperidon-3-yl, 2-Oxochinolin-3-yl, 2-Thioxochinolin-3-yl, 2,3-Dimethyl-1-phenyl-3-pyrazolin-5-on--4-yl, 1,2,3-Trimethyl-3-pyrazolin-5-on-4-yl, 2,3-Dimethyl-3-pyrazolin-5--on-4-yl, 2,3-Dihydro-3-oxobenzoxazin-2-yl, 2,3-Dihydro-3-oxobenzthiazin--2-yl, 1-Methyl-2-oxoindolin-3-yl, 2-Oxoindolin-3-yl, 1-Methyl-4-methoxy--2(1H)-pyridin-3-yl.

$R^2$ in Formel I steht für unverzweigte oder verzweigte Alkylreste mit 1 bis 4 C-Atomen, wie für Methyl, Ethyl, n-Propyl, i-Propyl, n-Butyl, sec.--Butyl, i-Butyl, tert.-Butyl.

Reste für $R^3$ in Formel I sind Propargyl, Alkyl mit 1 bis 3 C-Atomen, Alkenyl mit 3 oder 4 C-Atomen oder Halogenalkenyl mit 3 oder 4 C-Atomen, das bis zu drei Halogensubstituenten enthalten kann, beispielsweise Methyl, Ethyl, n-Propyl, i-Propyl, n-Butyl, sec.-Butyl, i-Butyl, tert.-Butyl, Allyl, 1-Chlorprop-1-en-3-yl, 2-Chlorprop-1-en-3-yl, 1,3-Dichlorprop-1-en-3-yl, 1,1,2-Trichlorprop-1-en-3-yl.

Als Salze der Verbindungen der Formel I kommen beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze sowie Ammonium- und Phosphoniumsalze, wie Tetraalkylammoniumsalze, Benzyltrialkylammoniumsalze, Tetraalkylphosphoniumsalze sowie Trialkylsulfonium- bzw. Trialkylsulfoxoniumsalze, in Betracht.

Bevorzugte Verbindungen der Formel I sind solche, bei denen $R^1$ Wasserstoff bedeutet und solche, bei denen A ein durch Alkyl mit bis zu 4 C-Atomen, insbesondere Methyl, oder Phenyl substituierten 2(1H)-Pyridinthionylrest ist, sowie Salze dieser Verbindungen.

Die Verbindungen der Formel I können durch Umsetzung von Verbindungen der Formel

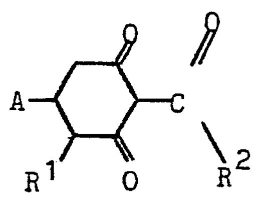

(II),

in der A, $R^1$ und $R^2$ die obengenannten Bedeutungen haben, mit Hydroxylaminderivaten $R^3O-NH_3Y$, in der $R^3$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80°C oder zwischen 0°C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 9, insbesondere von 4,5 bis 5,5. Die Einstellung des pH-Bereiches erfolgt

zweckmäßigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^3O-NH_3Y$, zugesetzt.

Als Lösungsmittel sind beispielsweise Dimethylsulfoxid, Alkohole, wie Methanol, Ethanol, Isopropanol, Benzol, Toluol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R^3O-NH_2$, in der $R^3$ die obengenannten Bedeutungen hat, in inerten Verdünnungsmitteln bei einer Temperatur zwischen 0° und dem Siedepunkt des Reaktionsgemisches, insbesondere zwischen 15 und 70°C, erhalten werden. Gegebenenfalls kann das Hydroxylamin als wäßrige Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril und cyclische Ether, wie Tetrahydrofuran.

Die Alkalimetallsalze der Verbindungen der Formel I können durch Behandeln dieser Verbindungen mit den entsprechenden Alkalihydroxiden, z.B. Natrium- oder Kaliumhydroxid, in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, z.B. die Mangan-, Kupfer-, Zink-, Eisen-, Calcium-, Magnesium- und Bariumsalze, können aus den Natriumsalzen durch Reaktion mit den entsprechenden Metallchloriden in wäßriger Lösung hergestellt werden. Ammonium-, Phosphonium-, Sulfonium- und Sulfoxoniumsalze können durch Umsetzung von Verbindungen der Formel I mit Ammonium-, Phosphonium-, Sulfonium- bzw. Sulfoxoniumhydroxiden gegebenenfalls in wäßriger Lösung hergestellt werden.

Die Verbindungen der Formel II können aus Cyclohexan-1,3-dionen der Formel III, die auch in den tautomeren Formen IIIa und IIIb vorliegen können,

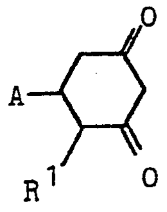

(III)

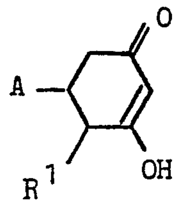

(III a)

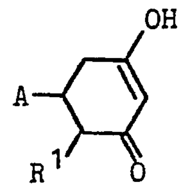

(III b)

nach literaturbekannten Methoden (Tetrahedron Letters, 29, 2491 (1975)) hergestellt werden.

Es ist auch möglich, Verbindungen der Formel II über die Zwischenstufe der Enolester, die bei der Umsetzung von Verbindungen der Formel III eventuell als Isomerengemische anfallen und in Gegenwart von Imidazol- oder Pyridinderivaten umgelagert werden (JP-OS 79/063052), herzustellen.

Zu den Verbindungen der Formel III gelangt man nach literaturbekannten Verfahren, wie dies aus folgendem Schema hervorgeht:

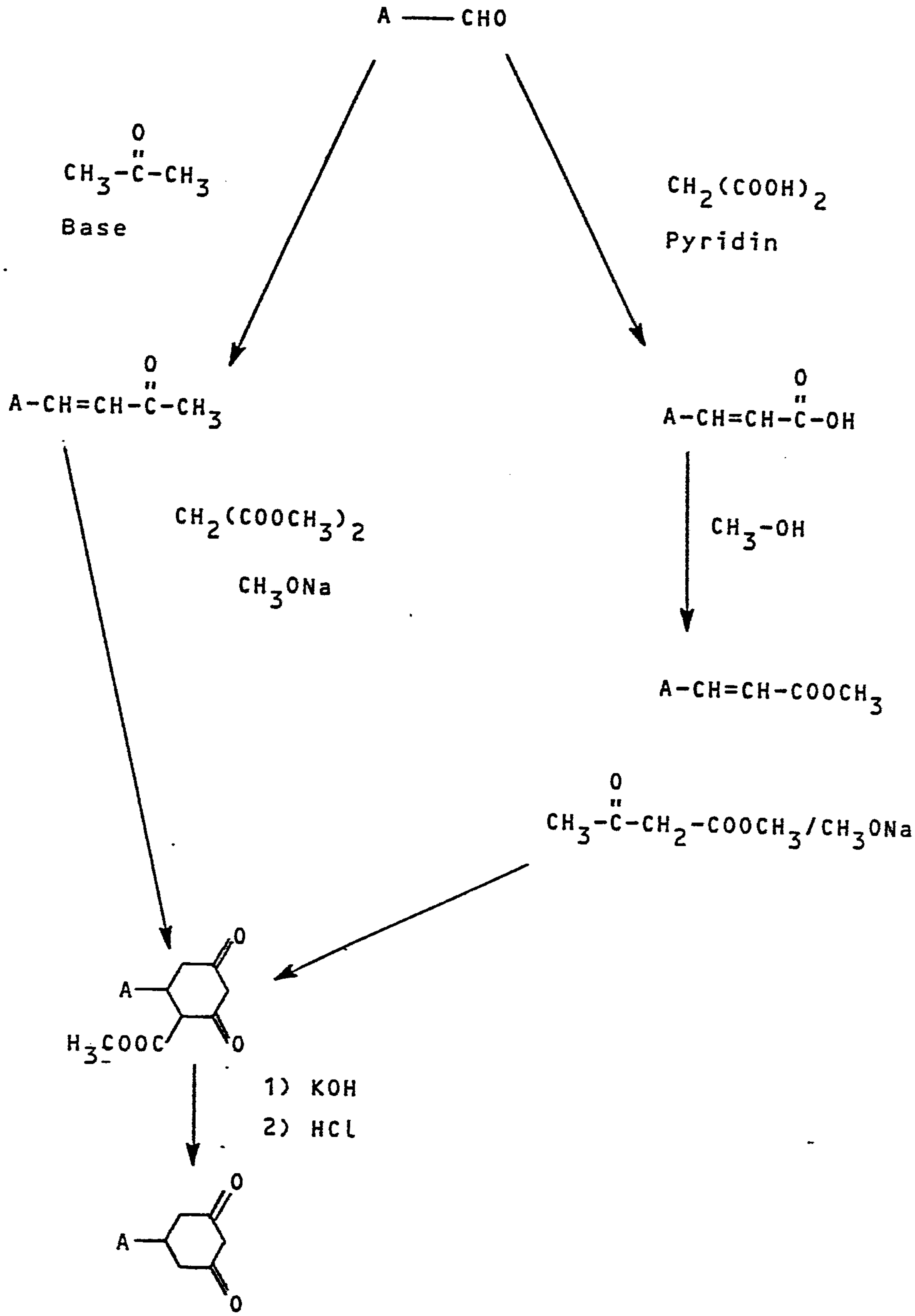
Base
Pyridin
1) KOH
2) HCl

0120448

Die Aldehyde A-CHO als Ausgangsverbindungen können nach literaturbekannten Verfahren, z.B. Vilsmeier-Formylierung, Oxidation von Alkoholen, Reduktion von Carbonsäurederivaten etc., hergestellt werden.

Die folgenden Beispiele erläutern die Herstellung der Cyclohexan-1,3-dion--derivate der Formel I. In den Beispielen verhalten sich Gewichtsteile zu Volumenteilen wie Kilogramm zu Liter.

Beispiel 1

Zu 6,1 Gewichtsteilen 2-Butyryl-5-(1-methyl-2(1H)-pyridinthion-3-yl)--cyclohexan-1,3-dion, gelöst in 100 Volumenteilen Methanol, werden 2,1 Gewichtsteile Ethoxyammoniumchlorid und 1,8 g Gewichtsteile Natriumhydrogencarbonat gegeben. Die Mischung wird 12 Stunden bei Raumtemperatur gerührt. Dann wird unter vermindertem Druck eingeengt, der Rückstand wird mit 100 Teilen Dichlormethan und 100 Teilen Wasser verrührt, die organische Phase wird abgetrennt, die wäßrige Phase wird mit 50 Dichlormethan extrahiert, die vereinigten organischen Phasen werden mit Wasser gewaschen, über Natriumsulfat getrocknet und unter vermindertem Druck eingeengt. Man erhält 2-(1-Ethoxyaminobutyliden)-5-(1-methyl-2(1H)-pyridinthion-3-yl)-cyclohexan-1,3-dion folgender Struktur: (Wirkstoff Nr. 1)

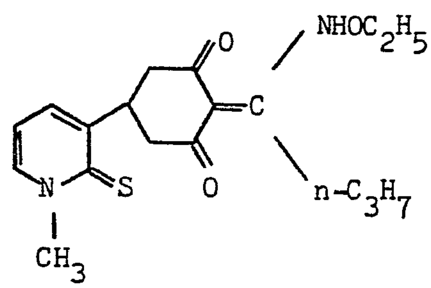

$C_{18}H_{24}N_2O_3S$ (348)
Ber.: C 62,04 H 6,94 N 8,04
Gef.: C 61,9 H 7,0 N 8,3
$^1$HNMR-Spektrum in $CDCl_3$ bezogen auf Tetramethylsilan als inneren Standard:
4,05 ppm $-O-CH_2$
3,90 ppm $N-CH_3$.

Beispiel 2

10 Gewichtsteile 2-Butyryl-5-(2-oxochinolin-3-yl)-cyclohexan-1,3-dion, 2,0 Gewichtsteile Ethoxyamin und 100 Teile Methanol werden bei Raumtemperatur 8 Stunden lang gerührt. Das Lösungsmittel wird unter vermindertem

Druck abdestilliert, der Rückstand in 200 Teilen Dichlormethan aufgenommen, die Lösung mit 5 %iger Salzsäure gewaschen, über Natriumsulfat getrocknet und unter vermindertem Druck eingeengt. Man erhält 2-(1-Ethoxyaminobutyliden)-5-(2-oxochinolin-3-yl)-cyclohexan-1,3-dion vom Fp. 155 bis 157°C. (Wirkstoff Nr. 2).

Die folgenden Verbindungen der Formel I erhält man in analoger Weise:

| Wirkstoff Nr. | A | $R^1$ | $R^2$ | $R^3$ | $n_D$/Fp (°C) |
|---|---|---|---|---|---|
| 3 | 2-Oxochinolin-3-yl | H | n-Propyl | Allyl | 190-193°C |
| 4 | 2-Oxochinolin-3-yl | H | n-Propyl | Propargyl | |
| 5 | 2-Oxochinolin-3-yl | H | n-Propyl | $-CH_2-CH=CHCl$ | |
| 6 | 1-Methyl-2(1H)-pyridinthion-3-yl | H | n-Propyl | Allyl | |
| 7 | 1-Phenyl-2(1H)-pyridinthion-3-yl | H | n-Propyl | Allyl | 130-133°C |
| 8 | 1-Phenyl-2(1H)-pyridinthion-3-yl | H | n-Propyl | Ethyl | 147-148°C |
| 9 | 2-Thioxochinolin-3-yl | H | n-Propyl | Ethyl | |
| 10 | 2-Thioxochinolin-3-yl | H | n-Propyl | Allyl | |
| 11 | 2(1H)-Pyridon-3-yl | H | n-Propyl | Allyl | |
| 12 | 2(1H)-Pyridon-3-yl | H | n-Propyl | Ethyl | |
| 13 | 2(1H)-Pyridon-3-yl | H | Ethyl | Ethyl | |
| 14 | 2(1H)-Pyridon-3-yl | H | Ethyl | Allyl | |
| 15 | 1-Phenyl-2(1H)-pyridon-3-yl | H | n-Propyl | Allyl | |
| 16 | 1-Phenyl-2(1H)-pyridon-3-yl | H | n-Propyl | Ethyl | |
| 17 | 1-tert.-Butyl-2(1H)-pyridon-3-yl | H | n-Propyl | Ethyl | |
| 18 | 1-tert.-Butyl-2(1H)-pyridon-3-yl | H | n-Propyl | Allyl | |
| 19 | 2(1H)-Pyridinthion-3-yl | H | n-Propyl | Allyl | |
| 20 | 2(1H)-Pyridinthion-3-yl | H | n-Propyl | Ethyl | |
| 21 | 1-tert.-Butyl-2(1H)-pyridinthion-3-yl | H | n-Propyl | Ethyl | |
| 22 | 1-tert.-Butyl-2(1H)-pyridinthion-3-yl | H | n-Propyl | Allyl | |
| 23 | 6-Methyl-2(1H)-pyridon-3-yl | H | n-Propyl | Allyl | |
| 24 | 6-Methyl-2(1H)-pyridon-3-yl | H | n-Propyl | Ethyl | |
| 25 | 4,6-Dimethyl-2(1H)-pyridon-3-yl | H | n-Propyl | Ethyl | |

| Wirkstoff Nr. | A | $R^1$ | $R^2$ | $R^3$ | $n_D$/Fp (°C) |
|---|---|---|---|---|---|
| 26 | 4,6-Dimethyl-2(1H)-pyridon-3-yl | H | n-Propyl | Allyl | |
| 27 | 4-Hydroxy-6-methyl-2(1H)-pyridon-3-yl | H | n-Propyl | Allyl | |
| 28 | 4-Hydroxy-6-methyl-2(1H)-pyridon-3-yl | H | n-Propyl | Ethyl | |
| 29 | 4-Methoxy-6-methyl-2(1H)-pyridon-3-yl | H | n-Propyl | Ethyl | |
| 30 | 4-Methoxy-6-methyl-2(1H)-pyridon-3-yl | H | n-Propyl | Allyl | |
| 31 | 1-Methyl-4-methoxy-2(1H)-pyridon-3-yl | H | n-Propyl | Allyl | |
| 32 | 1-Methyl-4-methoxy-2(1H)-pyridon-3-yl | H | n-Propyl | Ethyl | |
| 33 | 1-Ethyl-4-ethoxy-2(1H)-pyridon-3-yl | H | n-Propyl | Ethyl | |
| 34 | 1-Ethyl-4-ethoxy-2(1H)-pyridon-3-yl | H | n-Propyl | Allyl | |
| 35 | Piperidon-3-yl | H | n-Propyl | Allyl | |
| 36 | Piperidon-3-yl | H | n-Propyl | Ethyl | |
| 37 | 2,3-Dimethyl-1-phenyl-3--pyrazolin-5-on-4-yl | H | n-Propyl | Ethyl | |
| 38 | 2,3-Dimethyl-1-phenyl-3--pyrazolin-5-on-4-yl | H | n-Propyl | Allyl | |
| 39 | 2,3-Dimethyl-1-phenyl-3--pyrazolin-5-on-4-yl | Methoxy-carbonyl | n-Propyl | Ethyl | |
| 40 | 2,3-Dimethyl-1-phenyl-3--pyrazolin-5-on-4-yl | Methoxy-carbonyl | n-Propyl | Allyl | |
| 41 | 2,3-Dimethyl-1-phenyl-3--pyrazolin-5-on-4-yl | H | Ethyl | Allyl | |
| 42 | 2,3-Dimethyl-1-phenyl-3--pyrazolin-5-on-4-yl | H | Ethyl | Ethyl | |

| Wirkstoff Nr. | A | $R^1$ | $R^2$ | $R^3$ | $n_D$/Fp (°C) |
|---|---|---|---|---|---|
| 43 | 1,2,3-Trimethyl-3-pyrazolin--5-on-4-yl | H | n-Propyl | Ethyl | 132 |
| 44 | 1,2,3-Trimethyl-3-pyrazolin--5-on-4-yl | H | n-Propyl | Allyl | 98-100 |
| 45 | 2,3-Dimethyl-3-pyrazolin-5-on-4-yl | H | n-Propyl | Allyl | |
| 46 | 2,3-Dimethyl-3-pyrazolin-5-on-4-yl | H | n-Propyl | Ethyl | |
| 47 | 2,3-Dihydro-3-oxobenzoxazin-2-yl | H | n-Propyl | Ethyl | |
| 48 | 2,3-Dihydro-3-oxobenzoxazin-2-yl | H | n-Propyl | Allyl | |
| 49 | 1-Methyl-2-oxoindolin-3-yl | H | n-Propyl | Allyl | |
| 50 | 1-Methyl-2-oxoindolin-3-yl | H | n-Propyl | Ethyl | |
| 51 | Oxoindolin-3-yl | H | n-Propyl | Ethyl | |
| 52 | Oxoindolin-3-yl | H | n-Propyl | Allyl | |
| 53 | 1,2,3-Trimethyl-3-pyrazolin--5-on-4-yl | H | n-Propyl | Propargyl | |
| 54 | 2,3-Dimethyl-1-phenyl-3--pyrazolin-5-on-4-yl | H | n-Propyl | Propargyl | |

$^1H$-NMR-spektroskopische Daten bezogen auf Tetramethylsilan als innerem Standard in $CDCl_3$ als Lösungsmittel (s = Singulett, d = Dublett, t = Triplett, q = Quartett):

| Wirkstoff Nr. | Chemische Verschiebung [ppm] |
|---|---|
| 6 | 0,98 (t), 4,02 (s), 4,50 (d), 7.70 (d) |
| 24 | 1,00 (t), 2,38 (s), 6,08 (d) |
| 37 | 1,30 (t), 2,20 (s), 3,05 (s), 7,45 (m) |
| 38 | 2,20 (s), 4,55 (d), 7,45 (m) |
| 39 | 2,20 (s), 3,05 (s), 4,10 (q) |
| 41 | 1,0 (t), 2,20 (s), 3,0 (s), 4,55 (d), 7,45 (m) |
| 42 | 1,33 (t), 2,20 (s), 3,0 (s), 4,10 (q) |
| 43 | 2,10 (s), 3,20 (s), 4,05 (q) |
| 44 | 2,10 (s), 3,20 (s), 3,30 (s), 4,55 (d) |
| 53 | 1,0 (t), 2,12 (s), 3,22 (s), 4,70 (d) |
| 54 | 0,98 (t), 2,55 (d), 4,65 (d), 7,43 (m) |

Die Cyclohexan-1,3-dionderivate der Formel I und ihre Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser

homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 6 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 7 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 2 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 2 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 7 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 6 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,025 bis 3 kg/ha, vorzugsweise 0,1 bis 1,5 kg/ha.

Die Wirkung der Cyclohexan-1,3-dionderivate der Formel I und ihrer Salze auf das Wachstum von Pflanzen aus der Gräserfamilie (Gramineen) und breitblättrigen Kulturpflanzen läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelte sie danach. Die Sojapflanzen werden in einem mit Torfmull (peat) angereicherten Substrat angezogen. Zur Nachauflaufbehandlung werden entweder

direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmenge für die Nachauflaufbehandlung beträgt 0,25 kg bis 1,0 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Alopecurus myosuroides (Ackerfuchsschwanz), Avena fatua (Flughafer), Avena sativa (Hafer), Bromus spp. (Trespearten), Glycine max. (Sojabohnen), Lolium multiflorum (Ital. Raygras), Triticum aestivum (Weizen), Hordeum vulgare (Gerste), Setaria italica (Kolbenhirse), Beta vulgaris (Zuckerrüben), Gossypium hirsutum (Baumwolle), Sorghum bicolor (Kulturhirse), Sorghum halepense (Sudangras, Wilde Mohrenhirse), Zea Mays (Mais).

Bei Vorauflaufanwendung erweisen sich beispielsweise die Verbindungen Nr. 1, 2, 6 und 7 als herbizid wirksam gegen Pflanzen aus der Familie der Gräser.

Bei Nachauflaufversuchen bekämpft beispielsweise die Verbindung Nr. 1 unerwünschte Gräser selektiv in Kulturpflanzen, wie Soja und Weizen. Verbindung Nr. 6 ist ebenfalls selektiv in Getreide. Verbindung Nr. 2 ist auch im Nachauflaufverfahren wirksam gegen Grasarten, auch gegen gewisse Kulturgräser, ohne das Kulturgras Weizen zu schädigen. Breitblättrige Kulturpflanzen, wie Zuckerrüben, Soja und Baumwolle, werden ebenfalls nicht geschädigt.

In Anbetracht der Verträglichkeit der Applikationsmethoden können die erfindungsgemäßen Verbindungen noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |

| Botanischer Name | Deutscher Name |
|---|---|
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus duscis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Sasamum indicum | Sesam |
| Solanum tuberosum | Kartoffel |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |

Zur Verbreiterung des Wirkungsspektrums und zur Erzielung synergistischer Effekte können die Cyclohexan-1,3-dionderivate der Formel I und ihre Salze mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderi-

vate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate und andere in Betracht.

Außerdem kann es von Nutzen sein, die Cyclohexan-1,3-dionderivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Patentansprüche

1. Cyclohexan-1,3-dion-derivate der Formel

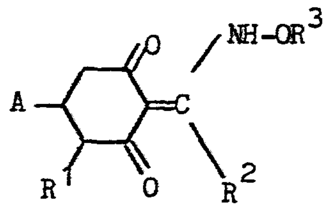

(I)

in der

A einen 4- bis 7-gliedrigen Heterocyclus mit 1 bis 3 Stickstoffatomen, der eine oder zwei Oxo- oder Thioxogruppen enthält und der gegebenenfalls ungesättigt, mit einem Aromaten kondensiert, mit maximal 3 Alkylresten mit bis zu 4 C-Atomen, dem Hydroxyrest, einem Alkoxyrest mit bis zu 4 C-Atomen oder einem gegebenenfalls substituierten Phenylrest substituiert sein kann,

$R^1$ Wasserstoff, Methoxycarbonyl, Ethoxycarbonyl, Methyl oder Cyano,

$R^2$ Alkyl mit 1 bis 4 C-Atomen und

$R^3$ Alkyl mit 1 bis 3 C-Atomen, Alkenyl mit 3 oder 4 C-Atomen, Halogenalkenyl mit 3 oder 4 C-Atomen und 1 bis 3 Halogensubstituenten oder Propargyl bedeuten,

und Salze dieser Verbindungen.

2. Cyclohexan-1,3-dionderivate der Formel I gemäß Anspruch 1, dadurch gekennzeichnet, daß $R^1$ Wasserstoff ist, und Salze dieser Verbindungen.

3. Cyclohexan-1,3-dionderivate der Formel I gemäß Anspruch 1, dadurch gekennzeichnet, daß A ein durch Alkyl mit bis zu 4 C-Atomen oder Phenyl substituierten 2(1H)-Pyridinthionylrest ist, und Salze dieser Verbindungen.

4. 2-(1-Ethoxyaminobutyliden)-5-(1-methyl-2(1H)-pyridinthion-3-yl)-cyclohexan-1,3-dion und Salze dieser Verbindung.

5. Verfahren zur Herstellung eines Cyclohexan-1,3-dionderivates der Formel I gemäß Anspruch 1, <u>dadurch gekennzeichnet</u>, daß man eine Verbindung der Formel

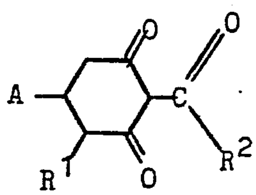

(II)

in der
A, $R^1$ und $R^2$ die im Anspruch 1 genannten Bedeutungen haben,

a) mit einer Ammoniumverbindung der Formel $R^3O-NH_3Y$, in der $R^3$ die im Anspruch 1 genannten Bedeutungen hat und Y ein Anion bedeutet, in einem inerten Verdünnungsmittel gegebenenfalls in Gegenwart von Wasser bei einer Temperatur zwischen 0 und 80°C in Gegenwart einer Base oder

b) mit einem gegebenenfalls in wäßriger Lösung vorliegenden Hydroxylamin der Formel $R^3O-NH_2$, in der $R^3$ die im Anspruch 1 genannten Bedeutungen hat, in einem inerten Lösungsmittel umsetzt.

6. Herbizid, enthaltend ein Cyclohexan-1,3-dionderivat der Formel I gemäß Anspruch 1 oder ein Salz einer solchen Verbindung.

7. Herbizid, enthaltend inerte Zusatzstoffe und ein Cyclohexan-1,3-dionderivat der Formel I gemäß Anspruch 1 oder ein Salz einer solchen Verbindung.

8. Herbizid, enthaltend inerte Zusatzstoffe und ein Cyclohexan-1,3-dionderivat der Formel I gemäß Anspruch 2 oder ein Salz einer solchen Verbindung.

9. Herbizid, enthaltend inerte Zusatzstoffe und ein Cyclohexan-1,3-dionderivat der Formel I gemäß Anspruch 3 oder ein Salz einer solchen Verbindung.

10. Verfahren zur Bekämpfung unerwünschten Pflanzenwachstums, <u>dadurch gekennzeichnet</u>, daß man die unerwünschten Pflanzen oder die von unerwünschten Pflanzenwachstum freizuhaltende Flächen mit einer herbizid wirksamen Menge eines Cyclohexan-1,3-dionderivates der Formel I gemäß Anspruch 1 behandelt.

Europäisches Patentamt

# EUROPÄISCHER RECHERCHENBERICHT

0120448
Nummer der Anmeldung

EP 84 10 3083

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 066 195 (BASF)<br>* Seiten 28-31 *<br>--- | 1,6 | C 07 D 213/70<br>A 01 N 43/34<br>A 01 N 43/38<br>A 01 N 43/72<br>C 07 D 215/22<br>C 07 D 215/36<br>C 07 D 213/64<br>C 07 D 213/69<br>C 07 D 211/74<br>C 07 D 231/26<br>C 07 D 231/20<br>C 07 D 265/02<br>C 07 D 209/34 |
| Y | EP-A-0 070 370 (BASF)<br>* Seiten 51-57 *<br>----- | 1,6 | |
| | | | **RECHERCHIERTE SACHGEBIETE (Int. Cl. 3)** |
| | | | C 07 D 213/00<br>C 07 D 215/00<br>C 07 D 211/00<br>C 07 D 231/00<br>C 07 D 209/00<br>C 07 D 265/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 18-06-1984 | VERHULST W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82